# EUROPEAN PATENT SPECIFICATION

(11) **EP 0 368 682 B1**
(45) Date of publication and mention of the grant of the patent: **31.05.1995**
(21) Application number: 89311701.0
(22) Date of filing: 10.11.1989
(51) Int. Cl.: A61K 9/18, A61K 9/16

(54) **Pharmaceutical ion exchange resin composition**
Pharmazeutische Ionenaustauscherharzzusammensetzung
Composition pharmaceutique à base d'une résine d'échange ionique

(30) Priority: 11.11.1988 GB 8826407; 07.12.1988 GB 8828592
(43) Date of publication of application: 16.05.1990
(73) Proprietor: Euroceltique S.A., Luxembourg (LU)
(72) Inventor: Malkowska, Sandra Therese Antoinette, Cambridge CB6 3RT (GB); Buxton, Ian Richard, Cambridge CB4 4HP (GB); Prater, Derek Allan, Cambridge CB4 4YS (GB); Norman, Alison Anne, By Huntingdon PE17 3D7 (GB)
(74) Representative: Lamb, John Baxter

(56) References cited:
- EP-A- 0 022 932
- EP-A- 0 122 219
- EP-A- 0 225 615
- DD-B- 249 634
- DE-A- 2 810 250
- FR-M- 792
- GB-A- 791 281
- US-A- 3 138 525
- P.H.LIST et al. "Hagers Handbuch der Pharmazeutischen Praxis", 4th edition,vol. 7, part B, 1977 SPRINGER VERLAG, Berlin, Heidelberg, New York pages 539-547
- Arzneiformenlehre, Wissenschaftliche Verlagsgesellschaft(1985), page 87
- List of Pharm Subst. 8th edition, Werbe-und Vertriebsgesellschaft Deutscher Apotheker m.b.H. p.100-101

## Description

The present invention relates to a pharmaceutical ion exchange resin composition and to a process of preparing the composition.

According to the present invention there is provided a process for the preparation of a pharmaceutical ion exchange resin composition that is readily dispersible in water comprising granulating an ion exchange resin having a pharmacologically active ingredient bound thereto with a sugar or a sugar alcohol in particulate form, in the presence of sufficient ware, aqueous alcohol or alcohol to facilitate granulation the amount of water, aqueous alcohol or alcohol employed in the granulation is from 1% to 20% (by weight) of the combined weight of the drug-resin complex and the sugar or the sugar alcohol.

EP-A2-0122219, FR-M-792, US-A-3138525, EP-A2-0225615 and GB-A-791281 all describe resin compositions in the form of standard tablet formulations and do not teach granule formulations for reconstitution. EP-A1-0022932 in Example 4 refers to granules prepared from hexoprenaline resinate, lactose, maize starch, collidone and glycerol palmistearate which are filled into capsules, there is no teaching of granulation with water, alcohol or aqueous alcohol as claimed in the present invention.

DE-A-2810250 and DD-B-249634 relate to pharmaceutical compositions containing cholestyramine, a polystyrene-divinylbenzene copolymer anion exchange resin. These references are directed to obtaining an acceptable cholestyramine resin formulation which is effective in binding reflexed bile.

GB 2218333A describes granules prepared by granulating a ranitidine resin adsorbate with a sugar alcohol (xylitol) using a solution of hydroxypropylmethyl cellulose in aqueous ethanol, there is no disclosure of the amount of granulating medium employed.

In the present specification, the phrase "readily dispersible in water" means that the composition must disperse, with stirring in 20 times its own weight of water within 10 seconds.

Any acidic or basic drug may be bound to the ion exchange resin. Preferably, however, active ingredients having a biological half life of 8 hours or less are used.

Suitable ingredients include
i. Narcotic analgesics, such as codeine, dihydrocodeine, hydromorphone, morphine, pentazocine and propoxyphene,
ii. Sympathomimetics, such as norephedrine and pseudoephedrine,
iii. Antitussives, such as dextromethorphan,
iv. Analgesics, such as aspirin,
v. Antiemetics, such as metoclopramide,
vi. Anticholinergics, such as atropine, ipratropium bromide and scopolamine,
vii. Muscle relaxants, such as cyclobenzaprine and papaverine,
viii. Bronchodilators, such as salbutamol, terbutaline and theophylline,
ix. Antibiotics, such as amoxycillin, ampicillin, azlocillin, bacampicillin, cefamandole, cefonicid, cefotaxime, cefotetan, cefoxitin, ceftriaxone, mezlocillin and piperacillin,
x. Antidepressants, such as bupropion, nomifensine, and nortriptyline,
xi. Antiasthmatics, such as cromolyn,
xii. Antineoplastics, such as tamoxifen.
xiii. Antiepileptics, such as valproic acid and phenytoin,
xiv. Cardiovascular agents, such as propranolol.

Acid addition salts or, if appropriate, alkali or alkaline earth metal salts of the above drugs would be particularly suitable for use in the present process.

Similarly, a wide range of cationic (for the basic drugs) or anionic (for the acidic drugs) exchange resins may be used to bind the active ingredient. Suitable ion exchange resins have acrylic, methacrylic, phenol-formaldehyde or dextran matrices. However, a preferred cationic ion exchange resin is a gel styrene-divinyl benzene sulphonic acid resin, such as Amberlite IR 120 (Trade Mark), Amberlite XE 69 (Trade mark) and Dowex 50W (Trade Mark), whilst a preferred anionic ion exchange resin is a gel styrene-divinyl benzene quaternary ammonium resin, such as Dowex SBR (Trade Mark) and Dowex SAR (Trade Mark).

The particle size and, if applicable, the degree of cross linking of the resin is determined by amongst other factors, the drug employed and the rate of drug release required. Preferably, however, the resin has a particle size of from 0.045 to 1mm, especially from 0.045 to 0.5mm. If applicable, the preferred degree of cross-linking is from 2% to 16%, especially from 8% to 12%.

The amount of drug bound to the resin is also determined by the choice of drug, as well as by the resin employed. Preferably the weight ratio of bound drug to resin is from 1:3 to 2:1, especially from 2:3 to 3:2.

Adsorption of the drug onto the ion exchange resin particles is a well known technique as shown in GB 824337, GB 1218102 and US 2990332 and demonstrated in the examples below. In general, the drug is mixed with an aqueous suspension of the resin and the complex is then dried. Adsorption of the drug onto the resin is detected by an assay of the suspending fluid.

Preferably the sugar or the sugar alcohol has a molecular weight of from 90 to 550, especially from 150 to 370. Suitable sugars/sugar alcohols are sucrose, dextrose, maltose, fructose, lactose, mannitol, sorbitol or, which is preferred, xylitol. The sugar/sugar alcohol is preferably finely divided, all of the sugar/sugar alcohol preferably having particle sizes of 0,6mm (600 microns) or less (30 mesh sieve). In a particularly preferred embodiment of the present process, at least 90% (by weight) of the sugar/sugar alcohol will have particle sizes of 0,25mm (250 microns) or less (60 mesh sieve).

The concentration of the sugar/sugar alcohol in the composition formed by the present process must be high enough to allow the composition to disperse readily in water. This means that there must be enough sugar/sugar alcohol present to allow the composition to disperse (within 10 seconds) when added with stirring to 20 times its weight of water. Generally, the composition will contain from 25% to 99%, preferably from 70% to 95%, (by weight) of the sugar/sugar alcohol.

The drug-resin complex and the sugar/sugar alcohol are granulated in the presence of sufficient water, aqueous alcohol or alcohol to facilate granulation. The most suitable alcohols are C₁-C₄ aliphatic alcohols, especially those having a boiling point, at 760mm Hg, of 100°C or less. Preferred alcohols are ethanol and isopropanol.

Preferably, the amount of granulating medium employed is from 1 to 20%, especially from 2 to 7%, by weight of the weight of the complex/sugar, sugar alcohol mix. In a particularly preferred embodiment of the present process the mixture is granulated until the particle size of the sugar/sugar alcohol matches the resin particle size. In the present specification, "matches" means that at least 80% (by wt) of the sugar/sugar alcohol has a particle size between 0.5 and 1.5 times the mean particle size of the drug-resin complex. Once the drug-resin complex and the sugar/sugar alcohol have been granulated, the granules formed are then dried, preferably, until their water content is below 3% (by weight) (when measured by the Karl Fischer method of moisture analysis).

Optionally, the drug-resin complex or the granules may be film coated with a material that permits release of the drug from the composition at a controlled rate.

The film coat will generally include a water insoluble material such as
(a) a wax, either alone or in admixture with a fatty alcohol,
(b) shellac or zein,
(c) a water insoluble cellulose derivative, especially ethyl cellulose,
(d) a polymethacrylate, especially Eudragit (Trade Mark).

Preferably, the film coat comprises a mixture of the water insoluble material and a water soluble material. The ratio of water insoluble to water soluble material is determined by, amongst other factors, the release rate required and the solubility characteristics of the materials selected.

The water soluble material may be, for example, triacetin, propylene glycol, polyethylene glycol, polyvinylpyrrolidone or, which is preferred, a water soluble cellulose, such as hydroxypropyl cellulose, or especially, hydroxypropylmethyl cellulose.

Suitable combinations of water insoluble and water soluble materials for the film coat include shellac and polyvinylpyrrolidone or, which is preferred, ethyl cellulose and hydroxypropylmethyl cellulose.

Once the above processing is complete, the composition may then be presented in a suitable dosage form, such as a capsule or a sachet. This is done simply by filling the capsule/sachet with the finished composition.

The present process and compositions prepared by the present process will now be described by way of example only,

### Example 1

Morphine Sulphate (Pentahydrate, 30gm), was added to purified water (EP, 110gm) and mixed until all of the powder was evenly wetted. With continuous mixing a cationic exchange resin (Dowex W50x8-200, Trade Mark, hydrous approx 50% (by wt) water, 60mg) was added to the morphine suspension. The stirring was continued for 24 hours at a rate that kept the resin suspended. The resin was then washed with purified water and dried in a fluid bed dryer.

Xylitol (500gm, 90% (by wt) having a particle size less than 200um) was mixed with the morphine-resin complex (40gm) and xanthan gum (20gm) in a granulator. With continuous mixing, purified water was added to the mixture until evenly wetted light granules were formed.

Generally the amount of water added was 2.0 - 5.0% (by wt) of the xylitol/complex/xanthan gum weight. The moist granules were then dried in a fluid bed dryer until the water content was below about 3.0% (by wt, Karl Fischer).

Each unit dose of this composition had the following formulation,

| | mg/unit dose |
|---|---|
| Morphine Sulphate (absorbed on the resin as morphine base) | 20 |
| Dowex W50x8-200 (Trade Mark) | 20 |
| Xylitol | 500 |
| Xanthan Gum | 20 |

### Example 2

The process of Example 1 was repeated to form a composition having the following formulation:

| | mg/unit dose |
|---|---|
| Metoclopramide Hydrochloride (absorbed on the resin as metoclopramide base) | 15 |
| Dowex W50x8-200 (Trade Mark) | 15 |
| Xylitol | 500 |
| Xanthan Gum | 20 |

### Example 3

The process of Example 1 was repeated to form a composition having the following formulation,

| | mg/unit dose |
|---|---|
| Hydromorphone Hydrochloride (absorbed on the resin as hydromorphone base) | 4 |
| Dowex W50x16-100 (Trade Mark) | 12 |
| Xylitol | 500 |
| Xanthan Gum | 20 |

### Example 4

The process of Example 1 was repeated to form a composition having the following formulation,

| | mg/unit dose |
|---|---|
| Theophylline Sodium (absorbed on the resin as theophylline base) | 100 |
| Dowex 2x8-200 (Trade Mark) | 200 |
| Xylitol | 870 |
| Xanthan Gum | 45 |
| Polyoxy 40-stearate (Trade Mark) | 5 |

### Comparative Example

The process of Example 1 was repeated except that the xylitol/morphine-resin complex/xanthan gum were dry mixed to form a powder, rather than wet granulated.

### Dispersibility of Resin Compositions in Water

Unit doses of the granular and powder products, produced according to Example 1 and the comparative Example respectively, were added to 10ml of water with continuous mixing.

The granular product immediately (within 10 seconds) formed an aqueous suspension of the resin. By contrast, the powder product formed a wetted mass that took a number of minutes to disperse in the aqueous medium (and thereby form a resin suspension).

Furthermore, whilst the granulated product gives a homogeneous product in which the drug substance is dispersed uniformly throughout the composition, the dry mixed powder was non-homogeneous, the drug substance being non-uniformly distributed throughout the composition.

## Claims

1. A process for the preparation of a pharmaceutical ion exchange resin composition that is readily dispersible in water comprising granulating an ion exchange resin, having a pharmacologically active ingredient bound thereto, with a sugar or a sugar alcohol in particulate form, in the presence of sufficient water, aqueous alcohol or alcohol to facilitate granulation, the amount of water, aqueous alcohol or alcohol employed in the granulation is from 1% to 20% (by weight) of the combined weight of the drug-resin complex and the sugar or the sugar alcohol.

2. A process according to claim 1 characterised in that the sugar or the sugar alcohol comprises sucrose, dextrose, maltose, fructose, lactose, mannitol, sorbitol or xylitol, preferably xylitol.

3. A process according to either claim 1 or claim 2 characterised in that all of the sugar or the sugar alcohol particles have a particle size of 0.6mm (600 microns) or less.

4. A process according to claim 3 characterised in that at least 90% (by wt) of the sugar or the sugar alcohol particles have a particle size of 0.25mm (250 microns) or less.

5. A process according to any of claims 1 to 4 characterised in that the amount of water, aqueous alcohol or alcohol employed in the granulation is from 2% to 7% (by weight) of the combined weight of the drug-resin complex and the sugar or the sugar alcohol.

6. A process according to any one of claims 1 to 5 characterised in that the granulation is continued until the particle size of the sugar or the sugar alcohol matches the particle size of the ion exchange resin.

7. A process according to any one of claims 1 to 6 characterised in that subsequent to the granulation, the granules are dried until their water content is below 3% (by weight).

8. A process according to any one of claims 1 to 7 characterised in that a capsule or a sachet is filled with the composition.

## Patentansprüche

1. Verfahren zur Herstellung einer in Wasser leicht dispergierbaren pharmazeutischen Ionenastauscherharz-Zusammensetzung, mit den Schritten: Granulieren eines Ionenaustauscherharzes, Binden eines pharmakologisch aktiven Bestandteils daran, mit einem Zucker oder einem Zuckeralkohol in Teilchenform, in Gegenwart von ausreichend Wasser, wäßrigem Alkohol oder Alkohol, um die Granulation zu erleichtern, wobei der beim Granulieren verwendete Anteil von Wasser, wäßrigem Alkohol oder Alkohol 1 Gew.-% bis 20 Gew.-% des Gesamtgewichts des Medikament-Harz-Komplexes und des Zuckers oder des Zuckeralkohols beträgt.

2. Verfahren nach Anspruch 1, dadurch gekennzeichnet, daß der Zucker oder Zuckeralkohol Sucrose, Dextrose, Maltose, Fructose, Lactose, Mannitol, Sorbitol oder Xylitol, vorzugsweise Xylitol aufweist.

3. Verfahren nach Anspruch 1 oder Anspruch 2, dadurch gekennzeichnet, daß alle Zucker- oder Zuckeralkoholteilchen eine Teilchengröße von 0,6 mm (600 »m) oder weniger aufweisen.

4. Verfahren nach Anspruch 3, dadurch gekennzeichnet, daß mindestens 90 Gew.-% der Zucker- oder Zuckeralkoholteilchen eine Teilchengröße von 0,25 mm (250 »m) oder weniger aufweisen.

5. Verfahren nach einem der Ansprüche 1 bis 4, dadurch gekennzeichnet, daß der bei der Granulation verwendete Anteil von Wasser, wäßrigem Alkohol oder Alkohol 2 Gew.-% bis 7 Gew.-% des Gesamtgewichts des Medikament-Harz-Komplexes und des Zuckers oder Zuckeralkohols beträgt.

6. Verfahren nach einem der Ansprüche 1 bis 5, dadurch gekennzeichnet, daß die Granulation fortgesetzt wird, bis die Teilchengröße des Zuckers oder des Zuckeralkohols der Teilchengröße des Ionenaustauscherharzes entspricht.

7. Verfahren nach einem der Ansprüche 1 bis 6, dadurch gekennzeichnet, daß nach der Granulation die Körner getrocknet werden, bis ihr Wassergehalt weniger als 3 Gew.-% beträgt.

8. Verfahren nach einem der Ansprüche 1 bis 7, dadurch gekennzeichnet, daß eine Kapsel oder ein Kissen mit der Zusammensetzung gefüllt werden.

## Revendications

1. Procédé pour la préparation d'une composition pharmaceutique de résine échangeuse d'ions qui est facilement dispersable dans l'eau comprenant la granulation d'une résine échangeuse d'ions ayant un ingrédient pharmacologiquement actif lié à celle-ci, avec un sucre ou un alcool de sucre sous forme particulaire en présence de suffisamment d'eau, d'alcool aqueux ou d'alcool pour faciliter la granulation, la quantité d'eau, d'alcool aqueux ou d'alcool employée dans la granulation étant de 1% à 20% (en poids) du poids de l'ensemble du complexe médicament-résine et du sucre ou de l'alcool de sucre.

2. Procédé selon la revendication 1, caractérisé en ce que le sucre ou l'alcool de sucre comprend le saccharose, le dextrose, le maltose, le fructose, le lactose, le mannitol, le sorbitol ou le xylitol, de préférence le xylitol.

3. Procédé selon l'une quelconque des revendications 1 et 2, caractérisé en ce que toutes les particules de sucre ou d'alcool de sucre ont une taille particulaire de 0,6 mm (600 microns) ou inférieure à 0,6 mm.

4. Procédé selon la revendication 3, caractérisé en ce qu'au moins 90% (en poids) des particules de sucre ou d'alcool de sucre ont une taille particulaire de 0,25 mm (250 microns) ou inférieure à 0,25 mm.

5. Procédé selon l'une quelconque des revendications 1 à 4, caractérisé en ce que la quantité d'eau, d'alcool aqueux ou d'alcool employée dans la granulation est comprise entre 2% et 7% (en poids) inclusivement du poids de l'ensemble du complexe médicament-résine et du sucre ou de l'alcool de sucre.

6. Procédé selon l'une quelconque des revendications 1 à 5, caractérisé en ce que la granulation est poursuivie jusqu'à ce que la taille particulaire du sucre ou de l'alcool de sucre s'équilibre avec la taille particulaire de la résine échangeuse d'ions.

7. Procédé selon l'une quelconque des revendications 1 à 6, caractérisé en ce que, après la granulation, les granules sont séchés jusqu'à ce que leur teneur en eau soit inférieure à 3% (en poids).

8. Procédé selon l'une quelconque des revendications 1 à 7, caractérisé en ce que la composition soit introduite dans une capsule ou un sachet .
